# EUROPÄISCHE PATENTSCHRIFT

(11) **EP 3 066 464 B1**
(45) Veröffentlichungstag und Bekanntmachung des Hinweises auf die Patenterteilung: **18.09.2019**
(21) Anmeldenummer: 14827405.3
(22) Anmeldetag: 05.11.2014
(51) Int. Cl.: G01N 31/22, A61B 10/02, A61B 10/00

(54) **TESTEINRICHTUNG ZUM DETEKTIEREN DER AN- ODER ABWESENHEIT EINES ANALYTEN IN EINER FLÜSSIGEN PROBE SOWIE VERFAHREN ZUR HERSTELLUNG**
TEST DEVICE FOR DETECTING THE PRESENCE OR ABSENCE OF AN ANALYTE IN A LIQUID SAMPLE, AND PRODUCTION METHOD
DISPOSITIF DE TEST POUR DETECTER LA PRESENCE OU ABSENCE D'UN ANALYTE DANS UN ECHANTILLON LIQUIDE ET PROCEDE POUR SA PREPARATION

(30) Priorität: 06.11.2013 AT 8522013
(43) Veröffentlichungstag der Anmeldung: 14.09.2016
(73) Patentinhaber: Joanneum Research Forschungsgesellschaft mbH, 8010 Graz (AT)
(72) Erfinder: MOHR, Gerhard, A-8720 Knittelfeld (AT)
(74) Vertreter: Strehl Schübel-Hopf & Partner
(86) Internationale Anmeldenummer: PCT/AT2014/000199
(87) Internationale Veröffentlichungsnummer: WO 2015/066740

(56) Entgegenhaltungen:
- WO-A2-02/30478
- GB-A- 2 408 330
- US-A- 6 117 090
- ARTUR J. MORO ET AL: "An ATP fluorescent chemosensor based on a Zn(II)-complexed dipicolylaminereceptor coupled with a naphthalimidechromophore-complexed dipicolylaminereceptor coupled with a naphthalimidechromophore", CHEMICAL COMMUNICATIONS, Bd. 46, Nr. 7, 1. Januar 2010 (2010-01-01) , Seiten 1085-1087, XP055180744, ISSN: 1359-7345, DOI: 10.1039/B919661G
- GERHARD J MOHR ET AL: "Design of acidochromic dyes for facile preparation of pH sensor layers", ANALYTICAL AND BIOANALYTICAL CHEMISTRY, SPRINGER, BERLIN, DE, Bd. 392, Nr. 7-8, 22. Oktober 2008 (2008-10-22), Seiten 1411-1418, XP019652967, ISSN: 1618-2650, DOI: 10.1007/S00216-008-2428-7
- TRUPP S ET AL: "Development of pH-sensitive indicator dyes for the preparation of micro-patterned optical sensor layers", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, Bd. 150, Nr. 1, 21. September 2010 (2010-09-21), Seiten 206-210, XP027278890, ISSN: 0925-4005 [gefunden am 2010-07-15]
- ANTJE KRILTZ ET AL: "Covalent immobilization of a fluorescent pH-sensitive naphthalimide dye in sol-gel films", JOURNAL OF SOL-GEL SCIENCE AND TECHNOLOGY, KLUWER ACADEMIC PUBLISHERS, BO, Bd. 63, Nr. 1, 7. April 2012 (2012-04-07), Seiten 23-29, XP035076187, ISSN: 1573-4846, DOI: 10.1007/S10971-012-2757-Z

## Beschreibung

Die vorliegende Erfindung bezieht sich auf eine Testeinrichtung zum schnellen Detektieren der An- oder Abwesenheit eines gasförmigen oder flüssigen Analyten in einer flüssigen Probe sowie zum Aufnehmen der flüssigen Probe, umfassend einen auf einem festen Träger aufgebrachten Indikatorfarbstoff sowie gegebenenfalls ein von dem Träger verschiedenes Halteelement sowie ein Verfahren zur Herstellung einer Testeinrichtung zum schnellen Detektieren der An- oder Abwesenheit eines Analyten in einer flüssigen Probe, bei welchem ein Indikatorfarbstoff auf einem festen Träger festgelegt wird sowie der feste Träger gegebenenfalls mit einem Halteelement verbunden wird.

Testeinrichtungen zu Detektieren der An- und Abwesenheit eines Analyten sowie Verfahren zu deren Herstellung sind seit längerem bekannt, wobei derartige Testeinrichtungen entweder in herkömmlicher Weise Indikatorstreifen sind, bei welchen auf einem festen Träger ein Indikatorfarbstoff festgelegt ist und bei welchen zum Detektieren eines Analyten der Indikatorstreifen in eine Testlösung eingetaucht wird, worauf ein Farbumschlag des auf dem Teststreifen festgelegten Indikatorfarbstoffs stattfindet.

Der EP 0 481 740 A2 ist hierbei ein pH-Mikrosensor zu entnehmen, bei welchem ein pHempfindliches Farbsensormaterial kovalent in ein Polyether-Polyisocyanate-Polymer gebunden ist.

Der GB 1 255 395 A sind nicht ausblutende Indikatorpapiere, Folien und Pulver zu entnehmen, bei welchen an eine Zellulosefaser ein oder mehrere Indikatorfarbestoffe kovalent gebunden vorliegen, um daraus Indikatorpapiere herzustellen.

Auch aus der JP 2006 308 423 A sowie der DE 4324991 A1 sind kovalent an ein Trägermaterial gebundene Indikatorfarbstoffe zu entnehmen.

Weiterhin sind auch Testeinrichtungen bekannt, bei welchen der in einer flüssigen Phase vorliegende Analyt durch mit der Testeinrichtung gekoppelte, saugfähige Träger aufgenommen wird und in weiterer Folge durch die Kapillarkräfte die flüssige Phase entlang des Testreifens entlang nach oben steigt und mit auf diesem Teststreifen festgelegten Indikatorfarbstoffen zur Reaktion gebracht wird, worauf aufgrund der Farbänderung die Anwesenheit eines Analyten bestätigt wird.

Nachteilig an derartigen Systemen ist, dass der Indikatorfarbstoff auf einem wenig saugfähigen Körper festgelegt ist, so dass die Indikatorteststreifen als solche nur eine sehr geringe Flüssigkeitsmenge aufnehmen können und insbesondere bei Vorliegen von geringen Konzentrationen eines Analyten in einer Probe häufig der Farbumschlag nicht oder nicht sehr deutlich erkannt wird, so dass ein eindeutiger Nachweis, ob ein bestimmter Analyt in einer flüssigen Probe vorliegt, häufig nicht oder nur sehr schlecht möglich ist.

Auch das ledigliche Tränken eines saugfähigen Körpers mit einem Indikatorfarbstoff und das nachfolgende Trocknen desselben erscheint nachteilig, da insbesondere beim Durchführen einer Analyse eines zu detektierenden Analyten die Gefahr besteht, dass der Indikatorfarbstoff in die Testlösung ausgewaschen wird, worauf jedenfalls ein Farbumschlag des Indikators auf dem Teststreifen und somit ein sinnvoller Nachweis eines zu untersuchenden Analyten nicht mehr möglich erscheint.

Die vorliegende Erfindung zielt nun darauf ab, die herkömmlichen Teststreifen mit den bekannten, saugfähigen Trägereinrichtungen, wie beispielsweise Wattestäbchen oder Pads zu kombinieren und eine Testeinrichtung zur Verfügung zu stellen, mit welcher es gleichzeitig gelingt, einen zu untersuchenden Analyten mit einem Träger aufzusaugen und auf demselben Träger zu detektieren. Weiterhin zielt die Erfindung darauf ab, ein Verfahren zur Herstellung einer derartigen Testeinrichtung zur Verfügung zu stellen.

Zur Lösung dieser Aufgaben ist eine erfindungsgemäße Testeinrichtung im Wesentlichen dadurch gekennzeichnet, dass der feste Träger aus einem aus sterilen saugfähigen Fasern, gewählt aus reinen oder gemischten Fasern aus Zellulose, Polyurethan-Hydrogelen, Poly(hydroxyethylmethacrylaten), Aminopolymeren, Polyacrylaten, Silikonen, Epoxiden, Sol-Gel Gläsern oder Polyolefinen komprimierten Körper besteht, dass auf dem Träger ein Indikatorfarbstoff kovalent gebunden vorliegt und dass der komprimierte Körper die flüssige Probe physikalisch bindet, insbesondere aufsaugt. Indem der feste Träger aus einem im Wesentlichen kugel-, zylinder- oder tropfenförmigen Körper, wie z.B. Pads, Tupfer oder Endbereichen von Wattestäbchen, aus Fasern gebildet wird, gelingt es, ausreichende Mengen einer einen Analyten enthaltenden Flüssigkeit in dem kugel-, zylinder- oder tropfenförmigen Körper, wie z.B. Pads, Tupfer oder Endbereichen von Wattestäbchen aufzunehmen und physikalisch zu binden, und indem auf diesem Träger ein Indikatorfarbstoff kovalent gebunden vorliegt, gelingt es gleichzeitig, den in der Flüssigkeit enthaltenen Analyten unmittelbar auf dem Träger durch das Auftreten eines Farbumschlags des Indikators zu detektieren. Weiterhin wird durch eine kovalente Anbindung der Indikatorfarbstoffe an die Fasern eine hohe Stabilität der Sensormaterialien erzielt, so dass keine Kontamination, insbesondere kein Ausbluten des Indikators erfolgt, beispielsweise von Patienten oder von Lebensmitteln, von wo Proben abgesaugt werden. Erfindungsgemäß werden Polymere, die mit Rezeptoren beladen sind, oder molekular geprägte Polymere für die Herstellung von Fasern eingesetzt werden, welche selektiv zur Erkennung von bestimmten Analyten beitragen können. In diesem Zusammenhang sind beispielsweise Polyesterfasern zu nennen, welches sich für Sauerstoffsensoren als besonders geeignet erwiesen haben. Weiterhin können die den Träger ausbildenden Fasern derart behandelt sein, dass beispielsweise Wirkstoffe daran adsorbiert sind oder darin festgelegt sind, so dass zusätzlich zu dem Effekt, dass die im Bereich einer zu detektierenden Substanz vorhandenen Flüssigkeiten aufgesaugt werden können und gasförmige oder flüssige Analyten in einer flüssigen Probe detektiert werden können, im Falle von einem medizinischen Einsatz beispielsweise Desinfektionsmittel und dgl. zusätzlich aufgebracht werden. Besonders wichtig erscheint in diesem Zusammenhang, dass Testergebnisse in wenigen Sekunden, wie 1 bis 20 s nach Kontaktieren der Wunde mit der Testeinrichtung erfolgt, so dass unmittelbar Verunreinigungen von Wunden, Entzündungsherde oder dgl. detektiert werden können. Hierdurch kann noch vor einem Verschluss der Wunde, z.B. durch eine Naht, mittels einer Kompresse oder dgl. eine entsprechende Behandlung vorgenommen werden und vor allem das Auftreten von Entzündungen vermieden werden.

Mit einer derartigen Vorgehensweise gelingt es, die Anwesenheit oder Abwesenheit eines gasförmigen oder flüssigen Analyten einerseits mit freiem Auge oder durch eine CCD Kamera oder ein Photometer festzustellen und überdies bleibt ein derartiger Farbumschlag für einen längeren Zeitraum bestehen, so dass eine Aussage über die Art und Menge des Analyten, der in der in dem Träger aufgenommenen Flüssigkeit enthalten war, möglich ist. So wäre es beispielsweise möglich, bei Patienten mit Wunden die Wundflüssigkeit zu entfernen und gleichzeitig eine rasche Information über den Wundstatus, z.B. den pH-Wert in der Wunde oder ob z.B. Gas, wie biogene Amine oder dgl. detektierbar ist, zu erhalten. Mit einer derartigen Vorgangsweise kann im Falle von auftretenden Problemen bei der Wundheilung zeitnah agiert werden und dadurch nachteilige Folgen für den Patienten und Kosten für die Wundheilung drastisch herabgesetzt werden.

Durch die erfindungsgemäße Verwendunng von molekular geprägten oder mit Rezeptormolekülen beladenen Fasern wird einerseits die Selektivität der Testeinrichtung erhöht und andererseits gelingt es mit einer derartigen Ausbildung, die Anreicherung des Analyten zu erreichen.

Indem, wie dies einer Weiterbildung der Erfindung entspricht, die Fasern zusätzlich mit Wirkstoffen, wie Desinfektionsmitteln und Medikamenten beladen sind, kann die erfindungsgemäße Testeinrichtung nicht nur die An- oder Abwesenheit eines gasförmigen oder flüssigen Analyten in einer flüssigen Probe testen, sondern zusätzlich beispielsweise in medizinischem Einsatz zur Wunddesinfektion oder zur Verabreichung von Medikamenten herangezogen werden.

Indem, wie dies einer Weiterbildung der Erfindung entspricht, der Träger aus gesponnenen und gegebenenfalls gebleichten Fasern oder Wirrfasern gebildet ist, gelingt es, einen Träger zur Verfügung zu stellen, welcher eine ausreichende Saugfähigkeit aufweist, um ausreichende Flüssigkeitsmengen aufzunehmen, um eine sichere und eindeutige Detektion eines Analyten zu ermöglichen. Weiterhin kann ein derartiger Träger auch problemlos sterilisiert werden, um z.B. im medizinischen Bereich zum Einsatz zu gelangen, oder mit Wirkstoffen, z.B. zur Blutstillung oder Desinfektion beladen werden.

Die Färbung mit Indikatorfarbstoffen kann sowohl nach Herstellung der Fasern (dem Spinnen) erfolgen als auch bereits vor der Herstellung der Fasern. Im ersten Fall werden die Fasern hergestellt und durch kovalentes Färben mit Indikatorfarbstoffen aus beispielsweise wässriger Lösung erhalten. Im zweiten Fall werden Nanopartikel, Mikropartikel oder Polymere kleinen Molekulargewichts, wie z.B. Aminocellulose oder Carboxymethcellulose mit einem Molekulargewicht von 50 bis 700 Millionen Dalton mit den Indikatorfarbstoffen kovalent gefärbt. Danach werden sie mit der gelösten Zellulose, beispielsweise als Xanthogenat oder als Lösung in N-Methyl-2-pyrrolidon oder in ionischen Flüssigkeiten vermischt bzw. dispergiert und danach zu Fasern versponnen.

Gemäß einer Weiterbildung der Erfindung ist in an sich bekannter Weise der Indikatorfarbstoff ein pH-Indikatorfarbstoff oder ein Redox-Indikatorfarbstoff oder anderer selektiver Indikatorfarbstoff. Sowohl pH-Indikatorfarbstoffe als auch Redox-Indikatorfarbstoffe können zum Nachverfolgen verschiedenster chemischer Reaktionen und somit für die unterschiedlichsten Einsatzzwecke angewandt werden. Mit selektiven Indikatorfarbstoffen können beispielsweise Sauerstoff, reaktive Sauerstoffspezies, wie Wasserstoffperoxid, CO, NO, CO₂, NO₂, Amine, Carbonsäuren, Ketone, Na⁺, K⁺, Ca₂⁺, Mg₂⁺, Ionen, Saccharide, Alkohole, Diole, Thiole, Stockoxide, Proteine, Metabolite, phosphororganischen Verbindungen, Nukleotide, DNA und Ameisensäure untersucht werden bzw. diese Verbindungen und Ionen nachverfolgt werden.

Die Selektivität und die Empfindlichkeit der Indikatoren werden speziell gesteuert und erfolgt über die verwendeten Materialien, wie z.B. Molekülstruktur der Indikatoren, die Eigenschaften der verwendeten Polymere und anderer Komponenten. Ein Beispiel zur Erfassung von Aminen ist die Trifluoracetylgruppe-Amin-Wechselwirkung. Rezeptoren, die eine sichtbare Änderung der Kontrollfläche für reaktive Sauerstoffspezies hervorrufen, können zum Beispiel Redoxsysteme sein, wobei häufig Metall-Ligand-Komplexe verwendet werden und die Erkennungsreaktion dann am Metallzentrum stattfindet. Metall-Ligand-Komplexe werden ebenso für die fluoreszente Detektion von Sauerstoff verwendet. Fluorescein-Sulfonatgruppen sind selektive Rezeptorgruppen für Wasserstoffperoxid, Maleimidfunktionen sind selektive Rezeptorgruppen für Thiole, Tricyanovinylgruppen sind selektive Rezeptorgruppen für primäre und sekundäre Amine, Pyrylium-Funktionen sind selektive Rezeptorgruppen für Aldehyde und Ketone, Aminogruppen sind selektive Rezeptorgruppen für Aldehyde und Ketone, Boronsäurefunktionen sind selektive Rezeptorgruppen für Diole, 1,2-Diaminobenzolgruppen sind selektive Rezeptorgruppen für Stickoxide, Aldehydfunktionen sind selektive Rezeptorgruppen für Aminosäuren.

Ein deutlich sichtbarer und glatter Farbumschlag kann gemäß der Erfindung dadurch gekennzeichnet sein, dass der Indikatorfarbstoff ein von gelb nach rot oder orange umschlagender Indikatorfarbstoff ist, insbesondere 2-((4-(2-Hydroxyethylsulfonyl)phenyl)diazenyl)-4-methoxyphenol, 4-Fluoro-2-((-4-(2-vinylsulfonyl)phenyl)diazenyl)phenol oder 4-Bromo-2-((4-(2-hydroxyethylsulfonyl)cyclohexa-1,4-dienyl)diazenyl)phenol. Auch Farbstoffe wie 1-Hydroxy-4-[4-(2-hydroxyethylsulfonyl)phenylazo]naphthalin-2-sulfonsäure-Kaliumsalz (Chromoionophore XVII), Dilithium(1+)ion 10 -amino-3-(vinylsulfonyl)-2,4-dioxo-3-azatri-cyclo[7.3.1.0^{5, 13}trideca-1(13),5,7,9,11-pentaen-7,11-disulfonat (Lucifer Gelb VS Dilithiumsalz), 4-Hydroxy-3-((4-(2-hydroxyethylsulfonyl)phenyl)diazenyl)benzonitril, 4-(Trifluoroacetyl)-4'-[N-(11-methacryloxyundecyl)-N-ethylamino]azobenzol, N-allyl-4-(N-methylpiperazinyl)-1,8-naphthalimid oder 4-[N,N-Bis(11-methacryloxyundecyl)-mino]-4'-(trifluoroacetyl)-stilben sind für eine kovalente Immobilisierung an saugfähige Fasern geeignet. Weiterhin sind auch Derivate der oben genannten Farbstoffe mit beispielsweise Azid- bzw. Ethingruppen, Maleimid- oder Carboxylgruppen, oder durch Dicyclohexylcarbodiimid oder Hydroxysuccinimidyl-Derivate aktivierte Carboxylgruppen zur kovalenten Anbindung geeignet.

Neben der Änderung der Absorption kann es auch zu einer Änderung der Reflektion, Brechungsindex, Oberflächenplasmonenresonanz, Infrarot- bzw. Ramanspektrum, Lumineszenz oder Lumineszenzabklingzeit kommen.

Indem die Fasern zu dem im Wesentlichen kugel-, zylinder- oder tropfenförmigen Körper verpresst sind, gelingt es einerseits, einen Träger zur Verfügung zu stellen, welcher ein gutes Oberflächen-zu-Volumen-Verhältnis aufweist und somit ausreichende Flüssigkeitsmengen aufnehmen kann, und andererseits eine relativ kleine Außenabmessung besitzt, um beispielsweise bei einem Einsatz im medizinischen Bereich auch an kleine Körperöffnungen bzw. Wunden herangeführt werden zu können. Hierbei sind vor allem Pads oder eine Art von Wattestäbchen bevorzugt.

Für eine Verdeutlichung des Nachweises eines zu untersuchenden Analyten ist das erfindungsgemäße Testmaterial dahingehend weitergebildet, dass der Indikatorfarbstoff an einer Außenoberfläche des Trägers festgelegt ist. Mit einer derartigen Ausbildung gelingt es, einen Farbumschlag des Indikatorfarbstoffs für einen Betrachter deutlich zu machen, ohne dass der gesamte Körper des Trägers mit Indikatorfarbstoffen dotiert sein muss. Durch eine derartige Vorgangsweise werden nicht nur die Kosten gesenkt, sondern überdies auch sichergestellt, dass ausreichende Flüssigkeitsmengen aufgenommen werden können, ohne dass große Mengen an Indikatorfarbstoff erforderlich wären, um einen deutlichen Farbumschlag des Indikators unmittelbar und klar zu erkennen.

Für einen einfachen Einsatz einer Testeinrichtung ist gemäß einer Weiterbildung der Erfindung das von dem Träger verschiedene Halteelement mit dem Träger mechanisch, insbesondere reibschlüssig verbunden. Durch eine derartige Art der Verbindung eines Halteelements mit dem Träger wird einerseits sichergestellt, dass die Testeinrichtung nicht durch beispielsweise einen Betätiger verunreinigt oder beschädigt wird, und andererseits sichergestellt, dass nicht Fremdstoffe, wie beispielsweise Kleber oder dgl. eine Beeinträchtigung der Testeinrichtung per se oder eines damit erzielten Testergebnisses mit sich bringen.

Eine mechanische bzw. reibschlüssige Verbindung einer Testeinrichtung mit einem Halteelement gelingt beispielsweise durch Verpressen des Trägers mit einem beispielsweise aus Karton, Holz oder aufgerautem Kunststoff bestehendem Halteelement oder auch durch ein Pressen des Trägers, z.B. in eine oder mehrere Vertiefungen des Halteelements, so dass Wattestäbchen oder Haltestreifen bzw. -bänder mit der Testeinrichtung verbunden werden.

Ein Verfahren zur Herstellung einer derartigen Testeinrichtung ist im Wesentlichen dadurch gekennzeichnet, dass an eine freie Oberfläche von Fasern Indikatorfarbstoffmoleküle aus einer insbesondere wässrigen Lösung mittels kovalenter Bindung festgelegt werden, dass nach einem insbesondere mehrmaligen Waschen der den Träger darstellenden Fasern mit den daran festgelegten Indikatormolekülen einem Verpressen im Wesentlichen zu kugel-, zylinder- oder tropfenförmigen, saugfähigen Körpern und einem nachfolgenden Sterilisieren und Trocknen unterworfen werden. Indem, wie dies der vorliegenden Erfindung entspricht, an eine freie Oberfläche von Fasern Indikatorfarbstoffmoleküle aus einer insbesondere wässrigen Lösung mittels kovalenter Bindung festgelegt werden, gelingt eine feste Verbindung des Indikatorfarbstoffs mit dem Träger, worauf bei einem Einsatz in der Praxis ein Auswaschen oder Ablösen des Indikators von den Trägermolekülen mit Sicherheit hintangehalten ist. Durch ein mehrmaliges Waschen der Fasern mit den daran festgelegten Indikatormolekülen werden jegliche überschüssigen Indikatormoleküle von dem Träger, insbesondere Zellulose entfernt, wodurch eine Verunreinigung, insbesondere ein Auswaschen der Indikatormoleküle in eine zu untersuchende Lösung der Flüssigkeit im praktischen Einsatz mit Sicherheit vermieden wird.

Durch ein Verpressen des Trägers zu im Wesentlichen kugel-, zylinder- und tropfenförmigen, saugfähigen Körpern gelingt es, ein gutes Oberflächen-zu-Volumen-Verhältnis zur Verfügung zu stellen, wodurch ausreichende Flüssigkeitsmengen durch den Träger aufgenommen werden können, um einen deutlichen Farbumschlag der daran festgelegten Indikatormoleküle zu gewährleisten. Weiterhin können z.B. bei Einsatz im medizinischen Bereich austretenden Körperflüssigkeiten, wie z.B. Wundsekret vollständig durch den Körper aufgenommen werden, um für einen Mediziner eine im Wesentlichen trockene Arbeitsumgebung für die Wundversorgung zu schaffen. Hierbei werden nicht nur trockene Arbeitsbedingungen für Mediziner geschaffen, sondern es gelingt in wenigen Sekunden, insbesondere 1 bis 20 s, vorzugsweise 2 bis 5 s einen Nachweis des Vorhandenseins bzw. Nichtvorhandenseins von Keimen, Verschmutzungen oder dgl. zu erhalten, so dass ein Mediziner vor einem Wundverschluss entsprechende Maßnahmen setzen kann und vor allem das Auftreten von Entzündungen so weit als möglich vermeiden kann.

Gemäß einer Weiterbildung der vorliegenden Erfindung ist das erfindungsgemäße Verfahren im Wesentlichen dadurch gekennzeichnet, dass die Fasern vor dem Festlegen der Indikatorfarbstoffmoleküle einem Spinnen und/oder Verwirren unterworfen werden. Durch eine derartige Vorgangsweise wird sichergestellt, dass nur lange Fasern mit beispielsweise einer Länge von bis zu 40 mm und einer Dicke von 10 bis 25 µm als Träger vorliegen, wodurch vermieden wird, dass beispielsweise bei Einsatz einer derartigen Testeinrichtung im medizinischen Bereich Fasern aus dem Träger in beispielsweise einem Wundbereich austreten können, wodurch Verunreinigungen oder Komplikationen bei der Wundheilung auftreten können. Indem die Fasern zu langen Fasern bzw. Garnen versponnen und/oder verwirrt werden, wird ein Austreten von kleinen Einzelfasern oder auch ein Disintegrieren des Trägers durch z.B. Aufquellen und Auswaschen der Fasern hintangehalten.

Indem, wie dies einer Weiterbildung der Erfindung entspricht, die Fasern mit den daran festgelegten Indikatorfarbstoffmolekülen auf einem Kern aus Fasern aufgebracht werden, wird einerseits die Menge an erforderlichen Indikatorfarbstoffmolekülen in einem derartigen Testelement bzw. einer derartigen Testeinrichtung so weit als möglich herabgesetzt und gleichzeitig sichergestellt, dass im praktischen Einsatz ein deutlicher Farbumschlag, welcher mit freiem Auge erkennbar ist, gewährleistet wird.

Es erübrigt sich festzuhalten, dass die den Kern des Trägers darstellende Fasern mit den mit den Indikatorfarbstoffmolekülen versetzten Fasern gleiche Eigenschaften aufweisen sollten, jedoch lediglich ein kovalentes Festlegen von Indikatorfarbstoffmolekülen nicht durchgeführt wurde.

Indem der Träger aus Fasern vor dem Trocknen an oder in einem Halteelement insbesondere reibschlüssig wird, gelingt es, eine Testeinrichtung zur Verfügung zu stellen, welche leicht mit der Hand ergriffen werden kann und bei welcher eine Verunreinigung des Trägers mit den daran festgelegten Indikatorfarbstoffmolekülen durch ein Ergreifen bzw. in Kontakt bringen mit gegebenenfalls verunreinigten Oberflächen jedenfalls vermieden werden kann.

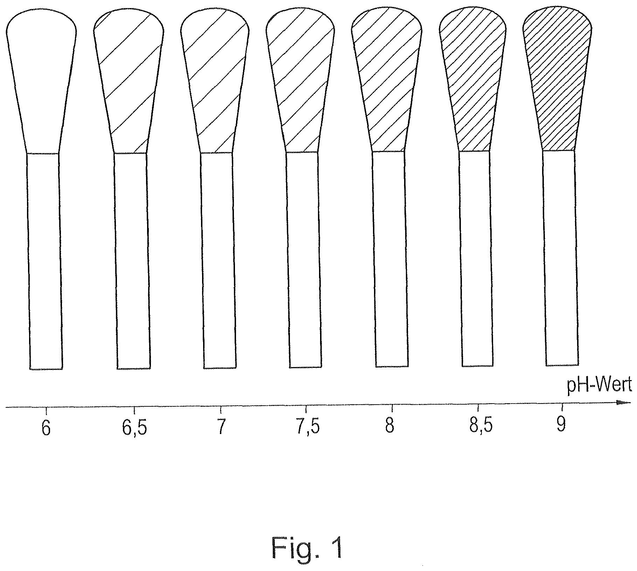

Die Erfindung wird nachfolgend anhand von Ausführungsbeispielen und Figuren näher erläutert. In diesen zeigen bei einer kovalenten Kopplung an Zellulosefasern
Beispiel 1: 1-Hydroxy-4-[4-(2-hydroxyethylsulfonyl)-phenylazo]naphthalin-2-sulfonsäure-Kaliumsalz einen Farbumschlag von orange nach violett.
Beispiel 2 und Fig. 1: Die Mischung von Tetrasodium (3Z)-5-amino-4-oxo-6-[4-(2-sulfonatooxyethylsulfonyl)-phenyl]diazenyl-2-[[4-(2-sulfonatooxyethylsulfonyl)phenyl]-hydrazinyliden]-naphthalen-2,7-disufonat mit 2-((4-(2-Hydroxyethylsulfonyl)phenyl)diazenyl)-4-methoxyphenol einen Farbumschlag von gelbgrün nach tiefrot.
Beispiel 3: 4-Fluro-2-((4-(2-hydroxyethylsulfonyl)phenyl)-diazenyl)phenol einen Farbumschlag von gelb nach rot.

In sämtlichen Beispielen wurde analog vorgegangen, wobei die kovalente Kopplung folgendermaßen erfolgt.

100 mg eines Indikatorfarbstoffs oder Farbstoffgemisches werden mit 1 g konzentrierter Schwefelsäure in einem Mörser gut durchmischt und 30 Minuten in einem Exsikkator stehen gelassen. Dadurch kann die 2-Hydroxyethylsulfonylgruppe des Indikatorfarbstoffs in ein reaktives Sulfonat umgewandelt werden. Diese Mischung wird dann in 900 ml Wasser geleert und mit 1,6 ml 32 % Natriumhydroxidlösung neutralisiert. Danach werden 25,0 g Natriumkarbonat in 100 ml Wasser und nachfolgend 5,3 ml einer 32 % Natriumhydroxidlösung zugeführt. Die Fasern werden in diese Färbelösung gelegt. Unter den basischen Bedingungen der Färbelösung wird das Farbstoffsulfonat in ein chemisch reaktives Vinylsulfonylderivat umgewandelt, welche sich kovalent an die Reaktivgruppen des Polymers (beispielsweise Hydroxylgruppen der Zellulose oder Aminogruppen des Polyurethans) anbindet. Auch der weitere Farbstoff bindet solchermaßen an die Reaktivgruppen des Polymers. Nach 60 Minuten werden die gefärbten Fasern aus dem Farbbad herausgenommen und mehrfach mit Wasser gewaschen. Danach werden die gefärbten Fasern an der Luft getrocknet.

Fig. 1 zeigt ein Beispiel einer deutlichen Farbänderung eines kovalent an Fasern von Wattestäbchen gebundenen Indikatorfarbstoffs. Hierbei ist der Farbumschlag von grün nach rot bei Erhöhung des pH-Werts gezeigt. Der Indikatorfarbstoff wird kovalent auf den Fasern von Wattestäbchen immobilisiert und die pH-Werte von links nach rechts betragen pH 6, pH 6.5, pH 7, pH 7.5, pH 8, pH 8.5, pH 9, resultierend in einer Farbänderung von grün nach rot, wobei die Farbe grün ohne Schraffierungen angedeutet ist und die Farbänderung durch die höhere Anzahl von Linien symbolisiert wird.

## Patentansprüche

1. Verfahren zur Herstellung einer Testeinrichtung zum Detektieren der An- oder Abwesenheit eines Analyten in einer flüssigen Probe, bei welchem ein Indikatorfarbstoff an einen festen Träger festgelegt wird sowie der feste Träger mit einem Halteelement verbunden wird, wobei an eine freie Oberfläche eines aus sterilen, saugfähigen Fasern aus Zellulose, Polyurethan-Hydrogelen, Poly(hydroxyethylmethacrylat), Aminopolymeren, Polyacrylaten, Silikonen, Epoxiden, Sol-Gel-Gläsern Polyolefinen oder Mischungen davon gebildeten Trägers Indikatorfarbstoffmoleküle aus einer wässrigen Lösung mittels kovalenter Bindung festgelegt werden, und die den Träger darstellenden Fasern mit den daran festgelegten Indikatormolekülen nach mehrmaligem Waschen einem Verpressen im Wesentlichen zu kugel-, zylinder- oder tropfenförmigen, saugfähigen Körpern und einem nachfolgenden Sterilisieren und Trocknen unterworfen werden, dadurch charakterisiert, dass die genannten Fasern molekular geprägt oder mit Rezeptormolekülen beladen sind.

2. Verfahren nach Anspruch 1, **dadurch gekennzeichnet, dass** die Fasern einem Spinnen und/oder Verwirren sowie gegebenenfalls einem Bleichen unterworfen werden.

3. Verfahren nach Anspruch 1 oder 2, **dadurch gekennzeichnet, dass** die Fasern zusätzlich mit Wirkstoffen wie Desinfektionsmitteln und Medikamenten beladen werden.

4. Verfahren nach Anspruch 1, 2 oder 3, **dadurch gekennzeichnet, dass** als Indikatorfarbestoff ein von gelb nach rot oder violett umschlagender Indikatorfarbstoff ist, insbesondere 2-((4-(2-Hydroxyethylsulfonyl)phenyl)diazenyl)-4-methoxyphenol, 4-Fluoro-2-((-4-(2-vinylsulfonyl)phenyl)diazenyl)phenol oder 4-Bromo-2-((4-(2-hydroxyethylsulfonyl)cyclohexa-1,4-dienyl)diazenyl)phenol, 1-Hydroxy-4-[4-(2-hydroxyethylsulfonyl)phenylazo]naphthalin-2-sulfonsäure-Kaliumsalz (Chromoionophore XVII), Dilithium(1+)ion 10-amino-3-(vinylsulfonyl)-2,4-dioxo-3-azatricyclo[7.3.1.0-{5, 13}trideca-1(13),5,7,9,11-pentan-7,11-disulfonat (Lucifer Gelb VS Dilithiumsalz), 4-Hydroxy-3-((4-(2-hydroxyethylsulfonyl)phenyl)diazenyl)benzonitril, 4-(Trifluoroacetyl)-4'-[N-(11-methacryloxyundecyl)-N-ethylamino]azobenzol, N-allyl-4-(N-methylpiperazinyl)-1,8-naphthalimid oder 4-[N,N-Bis(11-methacryloxyundecyl)-mino]-4'-(trifluoroacetyl)-stilben umschlagender Indikatorfarbestoff mittels kovalenter Bindung festgelegt wird.

5. Verfahren nach einem der Ansprüche 1 oder 4, **dadurch gekennzeichnet, dass** die Fasern mit den daran festgelegten Indikatorfarbstoffmolekülen auf einem Kern aus saugfähigen Fasern aufgebracht werden.

6. Verfahren nach einem der Ansprüche 1 bis 5, **dadurch gekennzeichnet, dass** der feste Träger vor dem Trocknen an oder in einem Halteelement reibschlüssig befestigt wird.

7. Testeinrichtung zum Detektieren der An- oder Abwesenheit eines gasförmigen oder flüssigen Analyten in einer flüssigen Probe sowie zum Aufnehmen der flüssigen Probe, umfassend einen auf einem festen Träger aufgebrachten Indikatorfarbstoff wobei der feste Träger an einem Halteelement befestigt ist, und der feste Träger aus einem Formkörper von sterilen und saugfähigen Fasern besteht, die im Wesentlichen zu einem kugel-, zylinder-, oder tropfenförmigen Körper verpresst sind, wobei die genannten Fasern aus der Gruppe Zellulose, Polyurethan-Hydrogelen, Poly(hydroxyethylmethacrylaten), Aminopolymeren, Polyacrylaten, Silikonen, Epoxiden, Sol-Gel Gläsern oder Polyolefinen oder Mischungen davon ausgewählt sind, und wobei der genannte Indikatorfarbstoff kovalent an den Träger gebunden vorliegt, **dadurch gekennzeichnet, dass** die genannten Fasern molekular geprägt oder mit Rezeptormolekülen beladen sind.

8. Testeinrichtung nach Anspruch 7, **dadurch gekennzeichnet, dass** die Fasern zusätzlich mit Wirkstoffen wie Desinfektionsmitteln und Medikamenten beladen sind.

9. Testeinrichtung nach Anspruch 7, **dadurch gekennzeichnet, dass** der Träger aus gesponnenen und gebleichten Fasern oder Wirrfasern gebildet ist.

10. Testeinrichtung nach Anspruch 7 oder 8, **dadurch gekennzeichnet, dass** der Indikatorfarbstoff an einer Außenoberfläche des Trägers festgelegt ist.

11. Testeinrichtung nach einem der Ansprüche 7 bis 9, **dadurch gekennzeichnet, dass** das von dem Träger verschiedene Halteelement mit dem Träger reibschlüssig verbunden ist.

12. Testeinrichtung nach einem der Ansprüche 8 bis 11, **dadurch gekennzeichnet, dass** der Indikatorfarbstoff ein von gelb nach rot oder violett umschlagender Indikatorfarbstoff ist, ausgewählt aus der Gruppe 2-((4-(2-Hydroxyethylsulfonyl)phenyl)diazenyl)-4-methoxyphenol, 4-Fluoro-2-((-4-(2-vinylsulfonyl)phenyl)diazenyl)phenol oder 4-Bromo-2-((4-(2-hydroxyethylsulfonyl)cyclo-hexa-1,4-dienyl)diazenyl)phenol, 1-Hydroxy-4-[4-(2-hydroxyethylsulfonyl)phenylazo]naphthalin-2-sulfonsäure-Kaliumsalz (Chromoionophore XVII), Dilithium(1+)ion 10 -amino-3-(vinylsulfonyl)-2,4-dioxo-3-azatricyclo[7.3.1.0^{5, 13}trideca-1(13),5,7,9, 11-pentan-7,11-disulfonat (Lucifer Gelb VS Dilithiumsalz), 4-Hydroxy-3-((4-(2-hydroxyethylsulfonyl)phenyl)diazenyl)benzonitril, 4-(Trifluoroacetyl)-4'-[N-(11-methacryloxyundecyl)-N-ethylamino]azobenzol, N-allyl-4-(N-methylpiperazinyl)-1,8-naphthalimid oder 4-[N,N-Bis(11-methacryloxyundecyl)-mino]-4'-(trifluoroacetyl)-stilben.

## Claims

1. A process for the manufacture of a test device for the detection of the presence or the absence of an analyte in a liquid sample, wherein an indicator color is fixed to a solid support and the solid support is bonded to a holding element, wherein indicator dye molecules from an aqueous solution are fixed by means of covalent bonding to a free surface of a carrier formed from sterile, absorbent fibres of cellulose, polyurethane hydrogels, poly(hydroxyethylmethacrylate), aminopolymers, polyacrylates, silicones, epoxides, sol-gel glasses, polyolefins or mixtures thereof, and wherein the fibres constituting the carrier having the indicator molecules fixed thereto are subjected, after repeated washing, to pressing essentially to form spherical, cylindrical or drop-shaped absorbent bodies and to a subsequent sterilisation and drying, **characterized in that** said fibres are molecularly imprinted or loaded with receptor molecules.

2. The process according to claim 1, **characterized in that** the fibres are subjected to spinning and/or tangling and, where appropriate, to bleaching.

3. The process according to claim 1 or 2, **characterized in that** the fibres are additionally loaded with active ingredients such as disinfectants and medicines.

4. The process according to Claim 1, 2 or 3, **characterized in that** the indicator dye used is an indicator dye which changes from yellow to red or violet, in particular 2-((4-(2-hydroxyethylsulfonyl)phenyl)diazenyl)-4-methoxyphenol, 4-fluoro-2-((-4-(2-vinylsulfonyl)-phenyl)diazenyl)phenol or 4-bromo-2-((4-(2-hydroxyethylsulfo-nyl)cyclohexa-1,4-dienyl)-diazenyl)phenol, 1-hydroxy-4-[4-(2-hydroxyethylsulfonyl)phenyl-azo]naphthalene-2-sulfonic acid potassium salt (chromoionophore XVII), dilithium(1+)ion 10-amino-3-(vinylsulfonyl)-2,4-dioxo-3-azatricyclo[7.3.1.0-{5,13}trideca-1(13),5,7,9,11-penetan-7,11-disulfonate (Lucifer Yellow VS dilithium salt), 4-hydroxy-3-((4-(2-hydroxyethyl-sulfonyl)-phenyl)diazenyl)-benzonitrile, 4-(trifluoroacetyl)-4'-[N-(11-methacryloxyundecyl)-N-ethylamino]azobenzene, N-allyl-4-(N-methylpiperazinyl)-1 ,8-naphthalimide or 4-[N,N-bis(11-methacryloxyundecyl)-mino]-4'-(trifluoroacetyl)-stilbene, is fixed by means of covalent bonding.

5. The process according to any one of claims 1 or 4, **characterized in that** the fibers with the indicator dye molecules fixed thereto are applied to a core of absorbent fibers.

6. The procss according to any one of claims 1 to 5, **characterized in that** the solid support is fixed to or in a holding element by frictional engagement before drying.

7. A test device for detecting the presence or absence of a gas or liquid analyte in a liquid sample and for receiving the liquid sample, comprising a dye applied to a solid support and optionally a holding element other than the support, wherein the solid support is bonded to a holding element, and the solid support consists of a molded body of sterile and absorbent fibres, which have been essentially pressed into spherical, cylindrical or drop-shaped bodies, wherein said fibres are selected from the group of cellulose, polyurethane hydrogels, poly(hydroxyethylmethacrylates), aminopolymers, polyacrylates, silicones, epoxides, sol-gel glasses or polyolefins or mixtures thereof, and wherein said indicator dye is covalently bonded to the support, **characterized in that** said fibres are molecularly imprinted or loaded with receptor molecules.

8. The test device according to claim 7, **characterized in that** the fibres are additionally loaded with active substances such as disinfectants and medicaments.

9. The test device according to claim 7, **characterized in that** the carrier is formed from spun and optionally bleached fibres or random fibres.

10. The test device according to claim 7 or 8, **characterized in that** the indicator dye is fixed to an outer surface of the support

11. The test device according to any one of claims 7 to 9, **characterized in that** the holding element, which is different from the support, is connected to the support by friction.

12. The test device according to one of claims 8 to 11, **characterized in that** the indicator dye changes from yellow to red or violet and is selected from the group of 2-((4-(2-hydroxy-ethylsulfonyl)phenyl)diazenyl)-4-methoxyphenol, 4-fluoro-2-((-4-(2-vinylsulfonyl)phenyl)-diazenyl)phenol or 4-bromo-2-((4-(2-hydroxyethylsulfonyl)cyclohexa-1,4-dienyl)diazenyl)-phenol, 1-hydroxy-4-[4-(2-hydroxyethylsulfonyl)phenylazo]naphthalin-2-sulfonic acid potassium salt (Chromoionophore XVII), dilithium(1+)ion 10-amino-3-(vinylsulfonyl)-2,4-dioxo-3-azatricyclo[7.3.1.0-{5, 13}trideca-1(13),5,7,9,11-pentan-7,11-disulfonat (Lucifer Yellow VS dilithium salt), 4-hydroxy-3-((4-(2-hydroxyethylsulfonyl)phenyl)diazenyl-)benzonitrile, 4-(trifluoroacetyl)-4'-[N-(11-methacryloxyundecyl)-N-ethylamino]azobenzene, N-allyl-4-(N-methylpiperazinyl)-1,8-naphthalimide or 4-[N,N-bis(11-methacryloxyundecyl)-mino]-4'-(trifluoroacetyl)-stilbene.

## Revendications

1. Procédé de fabrication d'un dispositif d'essai pour la détection de la présence ou de l'absence d'un analyte dans un échantillon liquide, dans lequel on fixe un colorant indicateur sur un support solide et on assemble le support solide à un élément de retenue, dans lequel on fixe par une liaison covalente des molécules de colorant indicateur à partir d'une solution aqueuse sur une surface libre d'un support composé de fibres stériles aspirantes de cellulose, d'hydrogels de polyuréthane, de poly(méthacrylate d'hydroxyéthyle), d'aminopolymères, de polyacrylates, de silicones, d'époxydes, de verres sol-gel, de polyoléfines ou de mélanges de ceux-ci, et on soumet les fibres représentant le support avec les molécules fixées sur celles-ci, après un lavage répété, à une compression en des corps aspirants essentiellement en forme de billes, de cylindres ou de gouttes et ensuite à une stérilisation et à un séchage, **caractérisé en ce que** lesdites fibres sont marquées de façon moléculaire ou sont chargées de molécules de récepteurs.

2. Procédé selon la revendication 1, **caractérisé en ce que** l'on soumet les fibres à un filage et/ou à un enchevêtrement ainsi qu'éventuellement à un blanchiment.

3. Procédé selon une revendication 1 ou 2, **caractérisé en ce que** l'on charge en outre les fibres avec des substances actives comme des agents de désinfection et des médicaments.

4. Procédé selon une revendication 1, 2 ou 3, **caractérisé en ce que** l'on fixe au moyen d'une liaison covalente à titre de colorant indicateur un colorant indicateur virant du jaune au rouge ou au violet, en particulier du 2-((4-(2-hydroxyéthylsulfonyl)phényl)diazényl)-4-méthoxyphénol,
du 4-fluoro-2-((4-(2-vinylsulfonyl)phényl)diazényl) phénol ou
du 4-bromo-2-((4-2-hydroxyéthylsulfonyl)cyclohexa-1,4-diényl)diazényl)phénol),
du sel de potassium de l'acide 1-hydroxy-4-[4-(2-hydroxyéthylsulfonyl)phénylazo]naphtalin-2-sulfonique (Chromoionophore XVII),
du dilithium(1+)ion 10-amino-3-(vinylsulfonyl)-2,4-dioxo-3-azatricyclo[7.3.1.0-{5,13}tridéca-1(13), 5,7,9,11-pentane-7,11-disulfonate (Jaune Lucifer VS sel de dilithium),
du 4-hydroxy-3-((4-(2-hydroxyéthylsulfonyl)phényl) diazényl)benzonitrile,
du 4-(trifluoroacétyl)-4'-[N-(11-méthacryloxyundécyl)-N-éthylamino]azobenzol,
du N-allyl-4-(N-méthylpipérazinyl)-1,8-naphtalimide ou du 4-[N,N-Bis(11-méthacryloxyundécyl)-mino]-4'-trifluoro acétyl)-stilbène.

5. Procédé selon une des revendications 1 ou 4, **caractérisé en ce que** l'on dépose les fibres avec les molécules de colorant indicateur fixées sur celles-ci sur un noyau en fibres aspirantes.

6. Procédé selon l'une quelconque des revendications 1 à 5, **caractérisé en ce que** l'on fixe le support solide par friction avant le séchage sur ou dans un élément de retenue.

7. Dispositif d'essai pour la détection de la présence ou de l'absence d'un analyte gazeux ou liquide dans un échantillon liquide ainsi que pour la réception de l'échantillon liquide, comprenant un colorant indicateur déposé sur un support solide, dans lequel le support solide est fixé à un élément de retenue, et le support solide se compose d'un corps moulé en fibres stériles et aspirantes, qui sont comprimées en un corps essentiellement en forme de bille, de cylindre ou de goutte, dans lequel lesdites fibres sont sélectionnées dans le groupe comprenant la cellulose, les hydrogels de polyuréthane, les poly(hydroxyéthylméthacrylates), les aminopolymères, les polyacrylates, les silicones, les époxydes, les verres sol-gel ou les polyoléfines ou des mélanges de ceux-ci, et dans lequel ledit colorant indicateur lié par covalence est situé sur le support, **caractérisé en ce que** lesdites fibres sont marquées de façon moléculaire ou sont chargées de molécules de récepteurs.

8. Dispositif d'essai selon la revendication 7, **caractérisé en ce que** les fibres sont en outre chargées de substances actives comme des agents de désinfection et des médicaments.

9. Dispositif d'essai selon la revendication 7, **caractérisé en ce que** le support est formé de fibres filées et blanchies ou de fibres enchevêtrées.

10. Dispositif d'essai selon une revendication 7 ou 8, **caractérisé en ce que** le colorant indicateur est disposé sur une surface extérieure du support.

11. Dispositif d'essai selon l'une quelconque des revendications 7 à 9, **caractérisé en ce que** l'élément de maintien différent du support est assemblé par friction au support.

12. Dispositif d'essai selon l'une quelconque des revendications 8 à 11, **caractérisé en ce que** le colorant indicateur est un colorant indicateur virant du jaune au rouge ou au violet, sélectionné dans le groupe se composant
du 2-((4-(2-hydroxyéthylsulfonyl)phényl)diazényl)-4-méthoxyphénol,
du 4-fluoro-2-((4-(2-vinylsulfonyl)phényl)diazényl) phénol ou
du 4-bromo-2-((4-2-hydroxyéthylsulfonyl)cyclohexa-1,4-diényl)diazényl)phénol),
du sel de potassium de l'acide 1-hydroxy-4-[4-(2-hydroxyéthylsulfonyl)phénylazo]naphtalin-2-sulfonique (Chromoionophore XVII),
du dilithium(1+)ion 10-amino-3-(vinylsulfonyl)-2,4-dioxo-3-azatricyclo[7.3.1.0-{5,13}tridéca-1(13), 5,7,9,11-pentane-7,11-disulfonate (Jaune Lucifer VS sel de dilithium),
du 4-hydroxy-3-((4-(2-hydroxyéthylsulfonyl)phényl) diazényl)benzonitrile,
du 4-(trifluoroacétyl)-4'-[N-(11-méthacryloxyundécyl)-N-éthylamino]azobenzol,
du N-allyl-4-(N-méthylpipérazinyl)-1,8-naphtalimide ou du 4-[N,N-Bis(11-méthacryloxyundécyl)-mino]-4'-trifluoro acétyl)-stilbène.
